# EUROPEAN PATENT APPLICATION

(11) **EP 4 537 825 A1**
(43) Date of publication of application: **16.04.2025**
(21) Application number: 23819058.1
(22) Date of filing: 05.06.2023
(51) Int. Cl.: A61K 31/47, A61K 31/55, A61K 31/4709, A61P 31/04, A61P 31/00

(54) **USE OF NITROXOLINE COMPOUND IN ENHANCING EFFECT OF ANTIBACTERIAL AGENT**

(30) Priority: 07.06.2022 CN 202210639706
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: DENG, Yijun, Shanghai 201203 (CN); LIN, Feng, Shanghai 201203 (CN); WU, Liang, Shanghai 201203 (CN)
(74) Representative: Regimbeau
(86) International application number: PCT/CN2023/098251
(87) International publication number: WO 2023/236893

(57) **Abstract**

The present invention relates to use of a nitroxoline compound in enhancing the effect of an antibacterial agent. In particular, the present invention relates to use of a compound represented by formula (I) or a pharmaceutically acceptable salt thereof in enhancing the antibacterial effect of the antibacterial agent. When the compound represented by formula (I) of the present invention is used in combination with the antibacterial agent, a synergistic or additive effect can be produced on the antibacterial agent, thereby enhancing the antibacterial effect, particularly for the antibacterial effect of a multi-medicament medicament-resistant strain.

## Description

### FIELD OF THE INVENTION

The present invention relates to a use of a nitroxoline compound of formula (I) or a pharmaceutically acceptable salt thereof for enhancing the antibacterial effect of an antibacterial agent, and a use of a nitroxoline compound of formula (I) or a pharmaceutically acceptable salt thereof in combination with an antibacterial agent for treating bacterial infectious diseases.

### BACKGROUND OF THE INVENTION

Infectious diseases are common and frequently occurring diseases that threaten human life and health, and are also one of the important complications and causes of death of multi-organ diseases. The use of anti-infective drugs has greatly reduced deaths from infectious diseases. Commonly used anti-infective drugs include β-lactam antibiotics, quinolone antibiotics, aminoglycoside antibiotics and the like, and their mechanisms of action include inhibiting synthesis of bacterial cell wall, inhibiting certain functions of cell membranes, inhibiting protein synthesis, inhibiting nucleic acid synthesis, and inhibiting folic acid synthesis.

At present, bacterial resistance is severe worldwide, and the problem of multidrug-resistant Gram-negative bacterial infection is serious. For example, *Escherichia coli, Pseudomonas aeruginosa,* and *Acinetobacter baumannii* that are resistant to carbapenem antibiotics are widely prevalent in clinical practice, and are difficult to treat or even untreatable.

Currently, there are two strategies to combat drug resistance, one is the discovery of new antimicrobial drug molecules, and the other is combination therapy.

However, for about two decades, a decrease in the number of new antimicrobial drug molecules entering the market has been observed. This decrease is largely due to high development costs and increased regulatory standards for approval.

It is conceivable that if another adjuvant is used in combination with existing antibiotics, it will be able to solve the problem that existing antibiotics alone are difficult to treat drug-resistant bacterial infections, which will greatly alleviate bacterial resistance and benefit all mankind.

### SUMMARY OF THE INVENTION

Therefore, in one aspect, the present invention relates to a pharmaceutical composition comprising a compound of formula (I) or a pharmaceutically acceptable salt thereof and at least one antibacterial agent as active ingredients, and a pharmaceutically acceptable carrier or excipient, 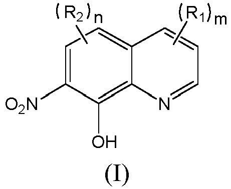 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
n is 0, 1 or 2;
m is 0, 1, 2 or 3.

The present invention also relates to a use of the pharmaceutical composition in the preparation of medicaments for the treatment of bacterial infectious diseases, especially multidrug-resistant bacterial infectious diseases.

In another aspect, the present invention relates to a use of a compound of formula (I) or a pharmaceutically acceptable salt thereof or a pharmaceutical composition comprising the same in the preparation of medicaments for enhancing the antibacterial effect of an antibacterial agent, 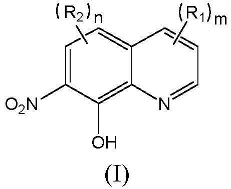 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
n is 0, 1 or 2;
m is 0, 1, 2 or 3.

In another aspect, the present invention relates to a use of a compound of formula (I) or a pharmaceutically acceptable salt thereof or a pharmaceutical composition comprising the same in combination with at least one antibacterial agent in the preparation of medicaments for enhancing the antibacterial effect of the antibacterial agent, 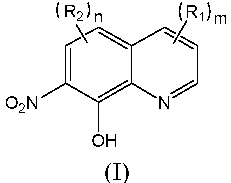 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
n is 0, 1 or 2;
m is 0, 1, 2 or 3.

The present invention also relates to a use of a compound of formula (I) or a pharmaceutically acceptable salt thereof or a pharmaceutical composition comprising the same in combination with at least one antibacterial agent in the preparation of medicaments for the treatment of bacterial infectious diseases, especially multidrug-resistant bacterial infectious diseases, 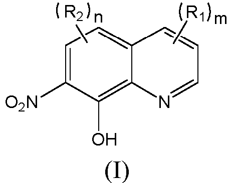 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl;
n is 0, 1 or 2;
m is 0, 1, 2 or 3.

In a preferred embodiment, in the compound of formula (I) of the present invention, R₁ is selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl.

In another preferred embodiment, in the compound of formula (I) of the present invention, R₁ is selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl.

In another preferred embodiment, in the compound of formula (I) of the present invention, R₁ is selected from the group consisting of hydrogen, halogen, C₁₋₆ alkyl, C₁₋₆ alkoxy and C₁₋₆ haloalkyl.

In a particularly preferred embodiment, in the compound of formula (I) of the present invention, R₁ is selected from hydrogen.

In another preferred embodiment, in the compound of formula (I) of the present invention, R₂ is selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl.

In another preferred embodiment, in the compound of formula (I) of the present invention, R₂ is selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl.

In another preferred embodiment, in the compound of formula (I) of the present invention, R₂ is selected from the group consisting of hydrogen, halogen, C₁₋₆ alkyl, C₁₋₆ alkoxy and C₁₋₆ haloalkyl.

In a particularly preferred embodiment, in the compound of formula (I) of the present invention, R₂ is halogen.

In a specific embodiment, in the compound of formula (I) of the present invention, n is 1 or 2.

In another specific embodiment, in the compound of formula (I) of the present invention, m is 0 or 1.

In a particularly preferred embodiment, in the compound of formula (I) of the present invention, each R₁ is independently hydrogen, each R₂ is independently halogen, n is 1 or 2, and m is 1.

In another preferred embodiment, the compound of formula (I) of the present invention is selected from the group consisting of:

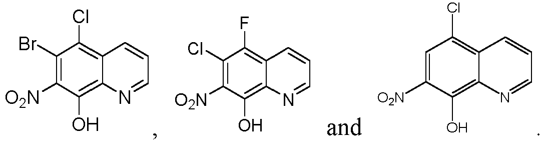

In another preferred embodiment, the antibacterial agent of the present invention is one or more selected from the group consisting of carbapenem antibiotics, polypeptide antibiotics, fluoroquinolone antibiotics, non-fluoroquinolone antibiotics, aminoglycoside antibiotics and glycopeptide antibiotics.

In a specific embodiment, the carbapenem antibiotic is meropenem.

In another specific embodiment, the polypeptide antibiotic is polymyxin.

In another specific embodiment, the fluoroquinolone antibiotic is levofloxacin.

In another specific embodiment, the non-fluoroquinolone antibiotic is nalidixic acid or nemonoxacin.

In another specific embodiment, the aminoglycoside antibiotic is gentamicin.

In another specific embodiment, the glycopeptide antibiotic is vancomycin.

In a preferred embodiment, the antibacterial agent of the present invention is selected from the group consisting of carbapenem antibiotics and polypeptide antibiotics.

In another specific embodiment, the antibacterial agent of the present invention is selected from the group consisting of polymyxin and meropenem.

In a preferred embodiment, the pharmaceutical composition according to the present invention is an antibacterial pharmaceutical composition.

In another preferred embodiment, the bacteria of the present invention is Gram-negative bacteria, such as *Escherichia coli, Pseudomonas aeruginosa* and *Acinetobacter baumannii*; preferably multidrug-resistant Gram-negative bacteria; more preferably multidrug-resistant bacteria resistant to carbapenem antibiotics, such as multidrug-resistant *Escherichia coli, Pseudomonas aeruginosa* and *Acinetobacter baumannii.*

In a specific embodiment, the bacteria of the present invention is selected from the strains of *Escherichia*; the strains of *Escherichia* is preferably *Escherichia coli,* wherein the *Escherichia coli* is non-resistant *Escherichia coli* and/or resistant *Escherichia coli,* preferably resistant *Escherichia coli,* and more preferably one or more of carbapenem-resistant *Escherichia coli,* quinolone-resistant *Escherichia coli,* aminoglycoside-resistant *Escherichia coli* and glycopeptide-resistant *Escherichia coli.*

In another specific embodiment, the bacteria of the present invention is preferably selected from the strains of *Acinetobacter*; the strains of *Acinetobacter* is preferably *Acinetobacter baumannii,* wherein the *Acinetobacter baumannii* is non-resistant *Acinetobacter baumannii* and/or resistant *Acinetobacter baumannii,* preferably resistant *Acinetobacter baumannii,* and more preferably carbapenem-resistant *Acinetobacter baumannii.*

In a specific embodiment, in the pharmaceutical composition or the use according to the present invention, the compound of formula (I) is 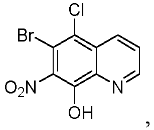 and the antibacterial agent is selected from the group consisting of meropenem and polymyxin.

In another specific embodiment, in the pharmaceutical composition or the use according to the present invention, the compound of formula (I) is 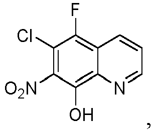 and the antibacterial agent is selected from the group consisting of polymyxin and meropenem, in particular polymyxin.

In another specific embodiment, in the pharmaceutical composition or the use according to the present invention, the compound of formula (I) is 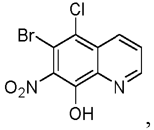 and the antibacterial agent is polymyxin.

In another specific embodiment, in the pharmaceutical composition or the use according to the present invention, the compound of formula (I) is 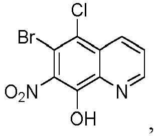 and the antibacterial agent is meropenem.

In another specific embodiment, in the pharmaceutical composition or the use according to the present invention, the compound of formula (I) is 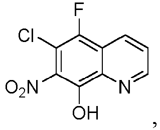 and the antibacterial agent is polymyxin.

In another specific embodiment, in the use according to the present invention, the compound of formula (I) is 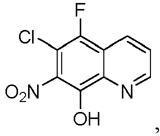 and the antibacterial agent is meropenem.

In another specific embodiment, in the pharmaceutical composition or the use according to the present invention, the compound of formula (I) is 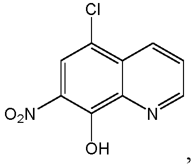 and the antibacterial agent is polymyxin.

In another specific embodiment, in the use according to the present invention, the compound of formula (I) is 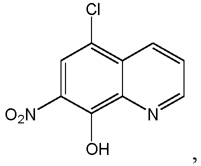 and the antibacterial agent is meropenem.

In a specific embodiment, in the use according to the present invention, the compound of formula (I) or the pharmaceutically acceptable salt thereof or the pharmaceutical composition comprising the same is administered simultaneously, successively or separately with the antibacterial agent.

The present invention further relates to a use of a compound or a pharmaceutically acceptable salt thereof or a pharmaceutical composition comprising the same in the preparation of medicaments for enhancing the antibacterial effect of an antibacterial agent, wherein the compound is selected from the group consisting of 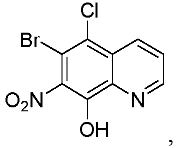 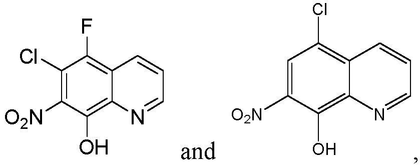 and the antibacterial agent is selected from the group consisting of polymyxin and meropenem.

The present invention also relates to a use of a compound in combination with an antibacterial agent in the preparation of medicaments for the treatment of bacterial infectious diseases, wherein the compound is selected from the group consisting of 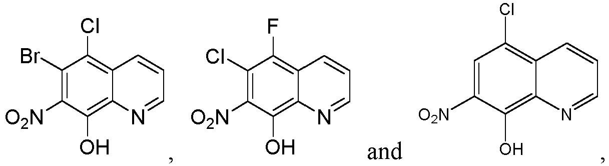 and the antibacterial agent is selected from the group consisting of polymyxin and meropenem, and the bacterial infectious disease is preferably a multidrug-resistant bacterial infectious disease.

The present invention further relates to a use of a combination of compound 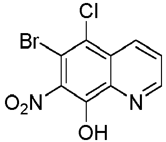 and polymyxin or meropenem in the preparation of medicaments for the treatment of bacterial infectious diseases; and the bacterial infectious disease is preferably multidrug-resistant bacterial infectious disease; the bacteria is preferably *Acinetobacter baumannii,* more preferably resistant *Acinetobacter baumannii,* and particularly preferably carbapenem-resistant *Acinetobacter baumannii.*

The present invention further relates to a use of a combination of compound 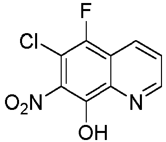 and polymyxin or meropenem in the preparation of medicaments for the treatment of bacterial infectious diseases; the bacterial infectious disease is preferably multidrug-resistant bacterial infectious disease; the bacteria is preferably *Acinetobacter baumannii,* more preferably resistant *Acinetobacter baumannii,* and particularly preferably *carbapenem*-resistant *Acinetobacter baumannii.*

The present invention further relates to a use of a combination of compound 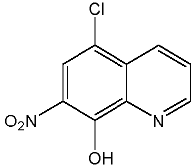 and meropenem in the preparation of medicaments for the treatment of bacterial infectious diseases; the bacterial infectious disease is preferably multidrug-resistant bacterial infectious disease; the bacteria is preferably *Acinetobacter baumannii* and *Escherichia coli,* more preferably resistant *Acinetobacter baumannii* and *Escherichia coli,* and particularly preferably carbapenem-resistant *Acinetobacter baumannii,* carbapenem-resistant *Escherichia coli,* quinolone-resistant *Escherichia coli,* aminoglycoside-resistant *Escherichia coli* and glycopeptide-resistant *Escherichia coli.*

The term "β-lactam antibiotics" refers to a large class of antibiotics with a β-lactam ring in their chemical structure, including the most commonly used penicillins and cephalosporins in clinical practice, as well as newly developed atypical β-lactam antibiotics such as cephalomycins, thiomycins, monobactams and the like.

The term "quinolone antibiotics" refers to synthetic antibacterial drugs containing the basic structure of 4-quinolone, such as floxacin, ofloxacin, ciprofloxacin, fleroxacin, etc.

The term "aminoglycoside antibiotics" refers to a class of antibiotics whose molecular structure comprises an aminocyclitol and one or more aminosugar molecules connected by glycosidic bonds to form a glycoside, such as streptomycin, gentamicin, tobramycin, kanamycin, amikacin, and the like.

The term "carbapenem antibiotics" has a structure similar to the penicillin ring of penicillins, except that the sulfur atom on the thiazole ring is replaced by carbon, and there is an unsaturated double bond between C₂ and C₃; in addition, its 6-hydroxyethyl side chain is in a trans conformation, such as imipenem, meropenem, panipenem, ertapenem, biapenem, doripenem, and the like.

The term "Gram-negative bacteria" refers to bacteria that show red color in a Gram-staining reaction, such as *Escherichia coli, Pseudomonas aeruginosa, Proteusbacillus vulgaris, Shigella dysenteriae, Klebsiella pneumoniae, Bacterium burgeri, Haemophilus influenzae, Haemophilus parainfluenzae, Moraxella catarrhalis, Acinetobacter, Yersinia, Legionella pneumophila, Bordetella pertussis, Bordetella parapertussis, Shigella, Pasteurella, Vibrio cholerae, Vibrio parahaemolyticus, Plesiomonas shigelloides,* and the like.

The term "multidrug resistance" refers to resistance to one drug and cross-resistance to multiple drugs with different structures and functions. "Multidrug-resistant bacteria" refers to a microorganism that is resistant to three or more classes of antimicrobial drugs with different mechanisms (such as aminoglycosides, macrolides, and β-lactams), rather than three of the same class.

The term "bacterial infectious disease(s)" refers to infectious diseases caused by bacteria invasion of the human body. Bacterial infections cover a wide range of areas and can be local infections or systemic infections. Local infection includes bacterial infection limited to one part, such as furuncle, carbuncle, pressure sore, and bacterial infection limited to one organ, such as pneumonia, gastritis, enteritis, dermatitis. Systemic infection includes bacteremia, sepsis, septicopyemia and the like. "Multidrug-resistant bacterial infectious disease(s)" refer to infectious diseases caused by multidrug-resistant bacteria invasion of the human body.

The polymyxin herein is polymyxin E, with a molecular formula of C₅₂H₉₈N₁₆O₁₃, and has antibacterial effects on Gram-negative bacteria such as *Pseudomonas aeruginosa, Escherichia coli, Klebsiella pneumoniae,* Haemophilus, *Enterobacter,* Salmonella, Shigella, *Bordetella pertussis,* Pasteurella and Vibrio. Polymyxin E is a basic polypeptide antibiotic produced by *Bacillus polymyxa colistin var,* which has a strong bactericidal effect on Gram-negative bacteria, and can treat animal feeding diseases caused by a variety of pathogens, and is characterized by good antibacterial spectrum, high efficiency and low toxicity.

Meropenem described herein, with the molecular formula of C₁₇H₂₅N₃O₅S, is an injectable antibiotic with a very broad antibacterial spectrum that is used to treat a variety of infections, including meningitis and pneumonia. It is a β-lactam antibiotic that belongs to the carbapenem class.

"Alkyl" refers to a saturated aliphatic hydrocarbon group, including straight and branched chain groups having the specified number of carbon atoms. Alkyl generally contains 1 to 20 carbon atoms (C₁-C₂₀ alkyl), preferably 1 to 12 carbon atoms (C₁-C₁₂ alkyl), more preferably 1 to 8 carbon atoms (C₁-C₈ alkyl) or 1 to 6 carbon atoms (C₁-C₆ alkyl) or 1 to 4 carbon atoms (C₁-C₄ alkyl). Non-limiting examples of alkyl include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. The alkyl can be substituted or unsubstituted. When substituted, the substituent group(s) can be substituted at any available connection point. Suitable optional substituents for alkyl include, but are not limited to, halogen, amino, nitro, cyano, hydroxy, thiol, carboxy, alkoxycarbonyl, oxo, thio, alkoxy, aryloxy, heteroaryloxy, haloalkyl, haloalkoxy, hydroxyalkyl, alkylamino, alkenyl, alkynyl, cycloalkyl, heterocyclyl, aryl, heteroaryl.

"Alkoxy" refers to an -O-(alkyl) group, wherein the alkyl is as defined above. Non-limiting examples of alkoxy include methoxy, ethoxy, propoxy, butoxy. The alkoxy can be optionally substituted or unsubstituted. When substituted, the substituent group(s) is preferably one or more group(s) independently selected from the group consisting of alkyl, alkenyl, alkynyl, alkoxy, haloalkyl, haloalkoxy, alkylthio, alkylamino, halogen, thiol, hydroxy, nitro, cyano, cycloalkyl, heterocyclyl, aryl, heteroaryl, cycloalkyloxy, heterocyclyloxy, cycloalkylthio, heterocyclylthio, carboxy and alkoxycarbonyl.

The term "haloalkyl" refers to an alkyl substituted by one or more halogen(s), wherein the alkyl is as defined above.

The term "haloalkoxy" refers to an alkoxy substituted by one or more halogen(s), wherein the alkoxy is as defined above.

The term "hydroxy" refers to an -OH group.

The term "halogen" refers to fluorine, chlorine, bromine or iodine.

The term "amino" refers to a -NH₂ group.

The term "thiol" refers to a -SH group.

A "pharmaceutical composition" refers to a mixture of one or more of the compounds according to the present invention or physiologically/pharmaceutically acceptable salts thereof with other chemical components, and other components such as physiologically/pharmaceutically acceptable carriers and excipients.

The term "pharmaceutically acceptable salt" refers to a salt of the compound of the present invention, which is safe and effective in mammals and has the desired biological activity.

According to conventional methods in the field of the present invention, the compound of the present invention can form a pharmaceutically acceptable base addition salt or acid addition salt with an alkali or an acid. The alkali includes inorganic alkali and organic alkali. Acceptable organic alkali includes diethanolamine, ethanolamine, N-methylglucamine, triethanolamine, tromethamine, and the like. Acceptable inorganic alkali includes aluminum hydroxide, calcium hydroxide, potassium hydroxide, sodium carbonate, sodium hydroxide, and the like. The acid includes inorganic acid and organic acid. Acceptable inorganic acid includes hydrochloric acid, sulfuric acid, nitric acid, phosphoric acid, hydrobromic acid, and the like. Acceptable organic acid includes acetic acid, trifluoroacetic acid, formic acid, ascorbic acid, and the like.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such composition can contain one or more ingredients selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the preparation of tablets. These excipients can be inert excipients, such as calcium carbonate, sodium carbonate, lactose, calcium phosphate or sodium phosphate; granulating and disintegrating agents, such as microcrystalline cellulose, cross-linked sodium carboxymethyl cellulose, corn starch or alginic acid; binders, such as starch, gelatin, polyvinylpyrrolidone or acacia; and lubricants, such as magnesium stearate, stearic acid or talc. The tablet can be uncoated or coated by means of known techniques which can mask drug taste or delay the disintegration and absorption of the active ingredient in the gastrointestinal tract, thereby providing sustained release over an extended period. For example, a water-soluble taste masking material can be used, such as hydroxypropyl methylcellulose or hydroxypropyl cellulose, or an extended release material can be used, such as ethyl cellulose, cellulose acetate butyrate.

An oral formulation can also be provided as hard gelatin capsules in which the active ingredient is mixed with an inert solid diluent, such as calcium carbonate, calcium phosphate or kaolin, or as soft gelatin capsules in which the active ingredient is mixed with a water-soluble carrier such as polyethylene glycol or an oil medium such as peanut oil, liquid paraffin or olive oil.

An aqueous suspension contains the active ingredient in admixture with an excipient suitable for the preparation of aqueous suspension. Such excipient is a suspending agent, such as sodium carboxymethylcellulose, methylcellulose, hydroxypropylmethylcellulose, sodium alginate, polyvinylpyrrolidone and acacia; a dispersant or humectant, which can be a naturally occurring phosphatide such as lecithin, or a condensation product of an alkylene oxide with fatty acid such as polyoxyethylene stearate, or a condensation product of ethylene oxide with a long chain aliphatic alcohol such as heptadecaethyleneoxy cetanol, or a condensation product of ethylene oxide with part esters derived from fatty acids and hexitols such as polyoxyethylene sorbitol monooleate, or a condensation product of ethylene oxide with partial esters derived from fatty acids and hexitol anhydrides such as polyoxyethylene sorbitan monooleate. The aqueous suspension can also contain one or more preservatives, such as ethylparaben or n-propylparaben, one or more colorants, one or more flavoring agents, and one or more sweeteners such as sucrose, saccharin or aspartame.

An oil suspension can be formulated by suspending the active ingredient in a vegetable oil such as peanut oil, olive oil, sesame oil or coconut oil, or in a mineral oil such as liquid paraffin. The oil suspension can contain a thickener, such as beeswax, hard paraffin or cetyl alcohol. The above sweetener and flavoring agent can be added to provide a palatable formulation. These compositions can be preserved by addition of an antioxidant, such as butylated hydroxyanisole or α-tocopherol.

The active ingredient in admixture with the dispersants or wetting agents, suspending agent or one or more preservatives can be prepared as a dispersible powder or granule suitable for the preparation of an aqueous suspension by addition of water. Suitable dispersants or wetting agents and suspending agents are as described above. Additional excipients, such as sweetening agents, flavoring agents and coloring agents, can also be added. These compositions are preserved by addition of an antioxidant such as ascorbic acid.

The pharmaceutical composition can also be in the form of an oil-in-water emulsion. The oil phase can be a vegetable oil such as olive oil or peanut oil, or a mineral oil such as liquid paraffin or a mixture thereof. Suitable emulsifying agent can be naturally occurring phosphatides, such as soy bean lecithin, and esters or partial esters derived from fatty acids and hexitol anhydrides such as sorbitan monooleate, and condensation products of said partial esters with ethylene oxide such as polyoxyethylene sorbitol monooleate. The emulsion can also contain a sweetener, flavoring agent, preservative and antioxidant. Syrup and elixir can be formulated with a sweetener, such as glycerol, propylene glycol, sorbitol or sucrose. Such formulations can also contain a moderator, a preservative, a colorant and an antioxidant.

The pharmaceutical composition can also be in the form of a sterile injectable aqueous solution. The acceptable vehicles and solvents that can be employed include water, Ringer's solution and isotonic sodium chloride solution. The sterile injectable formulation can be a sterile injectable oil-in-water microemulsion in which the active ingredient is dissolved in the oil phase. For example, the active ingredient can be firstly dissolved in a mixture of soybean oil and lecithin, the oil solution is then introduced into a mixture of water and glycerol and processed to form a microemulsion. The injectable solution or microemulsion can be introduced into a patient's bloodstream by local bolus injection. Alternatively, it can be advantageous to administrate the solution or microemulsion in such a way as to maintain a constant circulating concentration of the compound of the present invention. In order to maintain such a constant concentration, a continuous intravenous delivery device can be utilized.

The pharmaceutical composition can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent, such as a solution prepared in 1,3-butanediol. Moreover, sterile fixed oils can easily be used as a solvent or suspending medium. For this purpose, any blending fixed oils including synthetic mono- or di-glyceride can be employed. Moreover, fatty acids such as oleic acid can also be employed in the preparation of an injection.

It is well known to those skilled in the art that the dosage of a drug depends on a variety of factors including, but not limited to the following factors of activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the active compound or the type of pharmaceutically acceptable salt can be verified according to the traditional therapeutic regimens.

Compared with the prior art, the technical solution of the present invention has the following advantages: the compound of formula (I) or a pharmaceutically acceptable salt thereof is combined with an antibacterial agent, it exhibits a synergistic or additive effect on bacteria, especially multidrug-resistant bacteria, such as *Acinetobacter baumannii* when combined with an antibacterial agent, which makes the treatment of bacterial infection easier, and thus has a broad prospect of clinical application.

### DETAILED DESCRIPTION OF THE INVENTION

When there is no specifical definition, the technical terms in the present invention have the meanings commonly understood by those skilled in the art.

The present invention is further illustrated below by the Examples, but the present invention is not limited to the scope of the Examples.

The experimental methods in the following Examples without specifying specific conditions are conducted according to conventional methods and conditions, or according to the product specifications. The known reagents, solvents, and materials in the Examples can be synthesized by methods known in the art or are commercially available.

### Preparation Example 1: Preparation of 6-bromo-5-chloro-7-nitroquinolin-8-ol (1)

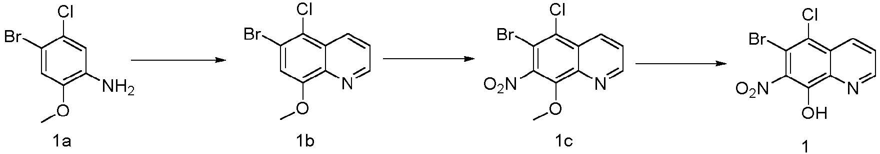

### Step 1: Preparation of 6-bromo-5-chloro-8-methoxyquinoline (1b)

4-Bromo-5-chloro-2-methoxyaniline (1a) (3.80 g, 16.1 mmol), sodium 3-nitrobenzene sulfonate (4.34 g, 19.3 mmol) and glycerol (2.96 mL, 40.2 mmol) were successively added to sulfuric acid (70 wt.%) (40.4 mL) at room temperature. The resulting mixture was stirred at 140°C for 4 hours, and cooled to room temperature. The pH of the aqueous phase was adjusted to about 8 to 9 with aqueous sodium hydroxide solution (1.0 M). The resulting mixture was extracted with dichloromethane (50.0 mL×3). The organic phases were combined, dried over anhydrous sodium sulfate, filtered, and concentrated to dryness under reduced pressure to obtain crude 6-bromo-5-chloro-8-methoxyquinoline (1b) (3.80 g, crude yield 87%).

### Step 2: Preparation of 6-bromo-5-chloro-8-methoxy-7-nitroquinoline (1c)

Nitric acid (65 wt.%) (8.4 mL, 198.0 mmol) was slowly added dropwise to a solution of crude 6-bromo-5-chloro-8-methoxyquinoline (1b) (1.80 g, 6.6 mmol) in acetic anhydride (30.0 mL) at 0°C, and stirred for 30 minutes. Sulfuric acid (98 wt.%) (1.20 mL, 22.6 mmol) was slowly added dropwise. The reaction mixture was stirred at 0°C for 30 minutes, then warmed up to room temperature and stirred for 48 hours, and then cooled to 0°C. The pH of the aqueous phase was adjusted to about 8 to 9 with aqueous sodium hydroxide solution (1.0 M). The resulting mixture was extracted with ethyl acetate (50.0 mL×3). The organic phases were combined, washed with saturated brine (50.0 mL), dried over anhydrous sodium sulfate, filtered, and concentrated under reduced pressure. The resulting residues were purified by silica gel column chromatography (gradient elution, eluent: petroleum ether/ethyl acetate=9/1-2/1) to obtain 6-bromo-5-chloro-8-methoxy-7-nitroquinoline (1c) (0.551 g, yield 24%).

### Step 3: Preparation of 6-bromo-5-chloro-7-nitroquinolin-8-ol (1)

6-Bromo-5-chloro-7-nitro-8-methoxyquinoline (1c) (1.65 g, 5.21 mmol) and lithium chloride (2.2 g, 52.4 mmol) were added to N,N-dimethylformamide (15.0 mL) at room temperature. The reaction mixture was stirred at 120°C for 1 hour, cooled to room temperature, and concentrated under reduced pressure to remove the organic solvent. Water (25.0 mL) was added to the resulting residues, stirred for 5 minutes, and filtered under reduced pressure. The filter cake was washed with water (5.0 mL×2), and dried under vacuum to obtain 6-bromo-5-chloro-7-nitroquinolin-8-ol (compound 1) (1.41 g, yield 89%).

¹H NMR (400 MHz, DMSO-*d*₆) δ: 8.70 (dd, *J =* 4.4, 1.6 Hz, 1 H), 8.41 (dd, *J =* 8.6, 1.4 Hz, 1 H), 7.68 (dd, *J =* 8.4, 4.0 Hz, 1 H).

MS calculated: 301.91, 303.91; MS found: 303.0, 305.0 [M+H]⁺.

### Preparation Example 2: Preparation of 6-chloro-5-fluoro-7-nitroquinolin-8-ol (2)

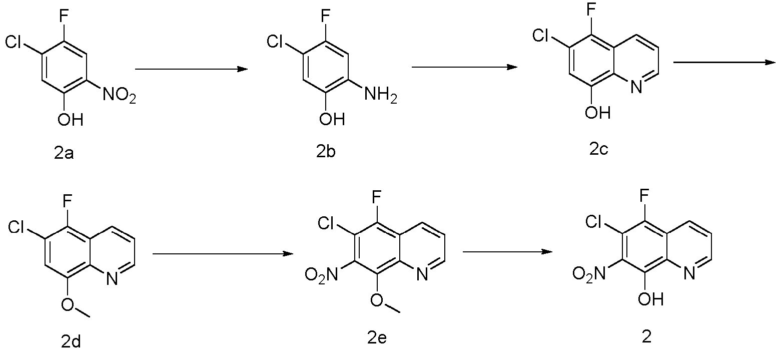

### Step 1: Preparation of 2-amino-4-fluoro-5-chlorophenol (2b)

2-Nitro-4-fluoro-5-chlorophenol (2a) (1.92 g, 10.0 mmol) was added to a mixture of tetrahydrofuran (60.0 mL) and water (60.0 mL) at room temperature. Sodium dithionite (85 wt %) (12.3 g, 60.0 mmol) was added in batches. The reaction mixture was stirred at room temperature for 1 hour, and extracted with ethyl acetate (30.0 mL×3). The organic phases were combined, washed with saturated brine (20.0 mL), dried over anhydrous sodium sulfate, filtered, and concentrated under reduced pressure. The resulting residues were purified by silica gel column chromatography (gradient elution, eluent: petroleum ether/ethyl acetate=100%/0 to 3/1) to obtain 2-amino-4-fluoro-5-chlorophenol (2b) (0.892 g, yield 55%).

### Step 2: Preparation of 5-fluoro-6-chloroquinolin-8-ol (2c)

2-Amino-4-fluoro-5-chlorophenol (2b) (0.892 g, 5.5 mmol), sodium 3-nitrobenzene sulfonate (1.48 g, 6.57 mmol) and glycerol (1.27 g, 13.8 mmol) were added successively to sulfuric acid (70 weight %) (8.0 mL) at room temperature. The resulting mixture was stirred at 140°C for 4 hours, and cooled to room temperature. The pH of the aqueous phase was adjusted to about 8 to 9 with aqueous sodium hydroxide solution (6.0 M). The resulting mixtures were extracted with dichloromethane (20.0 mL×3). The organic phases were combined, dried over anhydrous sodium sulfate, filtered, and concentrated under reduced pressure. The resulting residues were purified by silica gel column chromatography (gradient elution, eluent: petroleum ether/ethyl acetate=100%/0 to 7/1) to obtain 5-fluoro-6-chloroquinolin-8-ol (2c) (0.411 g, yield 38%).

### Step 3: Preparation of 5-fluoro-6-chloro-8-methoxyquinoline (2d)

Methyl iodide (0.17 mL, 2.73 mmol) was slowly added dropwise to a suspension of 5-fluoro-6-chloroquinolin-8-ol (2c) (0.411 g, 2.1 mmol) and potassium carbonate (0.58 g, 4.2 mmol) in N,N-dimethylformamide (6.0 mL) at room temperature,. The resulting mixture was stirred for 16 hours, and concentrated under reduced pressure to remove the organic solvent. Water (30.0 mL) was added to the resulting residues, and extracted with dichloromethane (10.0 mL×3). The organic phases were combined, washed with saturated brine (10.0 mL), dried over anhydrous sodium sulfate, filtered, and concentrated under reduced pressure. The resulting residues were purified by silica gel column chromatography (gradient elution, eluent: petroleum ether/ethyl acetate=100%/0 to 3/1) to obtain 5-fluoro-6-chloro-8-methoxyquinoline (2d) (0.412 g, yield 94%).

### Step 4: Preparation of 5-fluoro-6-chloro-7-nitro-8-methoxyquinoline (2e)

Nitric acid (65 weight %) (0.8 mL, 11.7 mmol) was slowly added dropwise to a solution of 5-fluoro-6-chloro-8-methoxyquinoline (2d) (0.412 g, 1.95 mmol) in acetic anhydride (10.0 mL) at 0°C, and stirred for 10 minutes. Sulfuric acid (98 weight %) (0.12 mL, 2.25 mmol) was slowly added dropwise. The reaction mixture was warmed up to room temperature, stirred for 16 hours, and cooled to 0°C. The pH of the aqueous phase was adjusted to about 8 to 9 with aqueous sodium hydroxide solution (6.0 M). The resulting mixtures were extracted with dichloromethane (20.0 mL×3). The organic phases were combined, washed with saturated brine (20.0 mL), dried over anhydrous sodium sulfate, filtered, and concentrated under reduced pressure. The resulting residues were purified by silica gel column chromatography (gradient elution, eluent: petroleum ether/ethyl acetate 100%/0 to 5/1) to obtain 5-fluoro-6-chloro-7-nitro-8-methoxyquinoline (2e) (84.0 mg, yield 17%).

### Step 5: Preparation of 5-fluoro-6-chloro-7-nitroquinolin-8-ol (2)

5-Fluoro-6-chloro-7-nitro-8-methoxyquinoline (2e) (84.0 mg, 0.39 mmol) and lithium chloride (162.0 mg, 3.9 mmol) were added to N,N-dimethylformamide (2.0 mL) at room temperature. The reaction mixture was stirred at 130°C for 1 hour, cooled to room temperature, and concentrated under reduced pressure to remove the organic solvent. Water (2.0 mL) was added to the resulting residues, stirred for 3 minutes, and filtered under reduced pressure. The resulting filter cake was washed with water (0.5 mL×2), and dried under vacuum to obtain 5-fluoro-6-chloro-7-nitroquinolin-8-ol (compound 2) (77.0 mg, yield 82%).

¹H NMR (400 MHz, DMSO-*d₆*) δ: 8.69 (d, *J =* 3.2 Hz, 1H), 8.25 (d, *J* = 8.3 Hz, 1H), 7.63 (dt, *J =* 12.8, 6.4 Hz, 1H).

MS calculated: 241.99; MS found: 243.1 [M+H]⁺.

### Preparation Example 3: Preparation of 5-chloro-7-nitro-8-hydroxyquinoline (compound A)

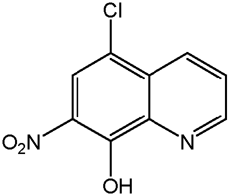

Compound A is a known compound, which was prepared according to the method of Example 1 in US20080312278A1.

### Test Example 1 Effect of compound 1 in combination with meropenem against drug-resistant Acinetobacter baumannii

This experiment is designed to determine the minimum inhibitory concentration (MIC) of compound 1 in combination with meropenem against *Acinetobacter baumannii* (a strain resistant to carbapenem antibiotics) and to calculate the fractional inhibitory concentration (FIC) index.

The materials and methods involved are as follows:

### 1. Test compound

| No. | Compound name | Batch number | Content wt% | Source |
|---|---|---|---|---|
| 1 | Compound 1 | DD20210033-08-P | 98.2 | Asieris Pharmaceuticals (Shanghai) Co., Ltd. |
| 2 | Meropenem | 130506-202004 | 86.8 | China National Institutes for Food and Drug Control |

### 2. Strains

Test bacteria: A total of 9 strains of *Acinetobacter baumannii,* all of which are resistant to carbapenem antibiotics (e.g., meropenem), are clinically isolated strains, and are provided by Shanghai Junji Medical Laboratory Co., Ltd.

### 3. Drug sensitivity test method

According to the recommendation of the Clinical and Laboratory Standards Institute (CLSI), the microbroth dilution method designed by the chessboard method was used to determine the minimum inhibitory concentration (MIC) of compound 1 in combination with meropenem against the above strains, and the fractional inhibitory concentration (FIC) index was calculated.

### (1) Formulation of antimicrobial drugs and preparation of drug sensitivity plates

Compound 1 was dissolved in dimethyl sulfoxide to obtain a stock solution I with a concentration of 1280 mg/L. The stock solution I was diluted to 512 mg/L with sterile water to obtain a gradient dilution first tube solution I. The gradient dilution first tube solution I was subjected to 1: 1 gradient dilution with sterile water to obtain a working solution I with a gradient range of 512 to 0.25 mg/L.

Meropenem was dissolved in sterile water to obtain a stock solution II with a concentration of 5120 mg/L. The stock solution II was diluted to 1024 mg/L with sterile water to obtain a gradient dilution first tube solution II. The gradient dilution first tube solution II was subjected to 1:1 gradient dilution with sterile water to obtain a working solution II with a gradient range of 1024 to 0.5 mg/L.

50 µL of solution was taken from each tube of working solution I, and added to the drug sensitivity plate row by row starting from the second well of each row (in each row, the concentration of compound 1 increased from the second well to the last well). 50 µL of solution was taken from each tube of working solution II, and added to the drug sensitivity plate column by column starting from the second well of each column (in each column, the concentration of meropenem increased from the second well to the last well). As a result, a chessboard cross with different concentrations of the two drugs was formed (wherein, there was neither compound 1 nor meropenem in the intersection well of the first row and the first column; the wells of the first row except the intersection well only contained compound 1, and the wells of the first column except the intersection well only contained meropenem; except for the above wells, the remaining wells contained compound 11 and meropenem). After the two drugs were mixed in equal volumes, the gradient ranges were reduced to 256 to 0.125 mg/L (compound 1) and 512 to 0.25 mg/L (meropenem), respectively.

According to the above process, the volume of the drug solution in each well containing only one drug of the drug sensitivity plate was 50 µL, thus 50 µL of sterile water was added to each well. 100 µL of sterile water was added to the intersection well of the first row and the first column. After that, 100 µL of bacterial solution (formulated according to step (3)) was added to all the wells of the drug sensitivity plate and mixed. Therefore, the gradient ranges were reduced to 128 to 0.06 mg/L (compound 1) and 256 to 0.125 mg/L (meropenem).
(2) The culture medium of the bacterial solution: Cation-Adjusted Mueller-Hinton Broth (CAMHB), a product of BBL, USA, batch number 9015952.
(3) Formulation method of bacterial solution: According to the direct colony suspension method, the test bacteria cultured overnight were prepared into a 0.5 McFarland turbidity tube using physiological saline. Then, the resulting solution was dilute 100 times with the above culture medium. 100 µL of the resulting bacterial solution was mixed well with the solution (100 µL) in each well of the drug sensitivity plate, and the final inoculation amount was 10⁵ CFU/mL.
(4) Culture conditions: Culturing at 35±2°C for 20 hours in air.

### 4. Results and evaluation

(1) The experimental results were determined according to the standard of CLSI 2019 M100 29^{th} edition.
(2) Calculation formula of fractional inhibitory concentration index: FIC = MIC of drug A in combination/MIC of drug A alone + MIC of drug B in combination/MIC of drug B alone; wherein, FIC≤0.5 indicates synergistic effect; 0.5<FIC≤1.0 indicates additive effect; 1<FIC≤2.0 indicates irrelevant effect; and FIC>2 indicates antagonistic effect.

The specific experimental results are shown in Table 1 below, wherein the *Acinetobacter baumannii* specifically refers to "carbapenem-resistant *Acinetobacter baumannii".*

**Table 1 FIC index of compound 1 in combination with meropenem**

| No. | Bacteria name | Strain No. | Compound 1 (mg/L) | | Meropenem (mg/L) | | FIC index | Results |
|---|---|---|---|---|---|---|---|---|
| | | | MIC alone | MIC in combination | MIC alone | MIC in combination | | |
| 1 | *Acinetobacter baumannii* | 18-J11-034 | 4 | 2 | 64 | 16 | 0.750 | Additive effect |
| 2 | *Acinetobacter baumannii* | 18-J15-030 | 16 | 8 | 64 | 8 | 0.625 | Additive effect |
| 3 | *Acinetobacter baumannii* | 18-J21-095 | 8 | 4 | 64 | 16 | 0.750 | Additive effect |
| 4 | *Acinetobacter baumannii* | 18-J24-015 | 8 | 2 | 64 | 32 | 0.750 | Additive effect |
| 5 | *Acinetobacter baumannii* | 18-J40-021 | 8 | 4 | 64 | 16 | 0.750 | Additive effect |
| 6 | *Acinetobacter baumannii* | 18-J51-052 | 4 | 2 | 32 | 4 | 0.625 | Additive effect |
| 7 | *Acinetobacter baumannii* | 18-J11-001 | 8 | 4 | 32 | 8 | 0.750 | Additive effect |
| 8 | *Acinetobacter baumannii* | 18-J11-097 | 8 | 4 | 64 | 16 | 0.750 | Additive effect |
| 9 | *Acinetobacter baumannii* | 18-J11-002 | 4 | 2 | 64 | 16 | 0.750 | Additive effect |

As can be seen from Table 1, the combination of compound 1 with meropenem can significantly reduce the MIC of meropenem, and has an additive effect on *Acinetobacter baumannii* (strains resistant to carbapenem antibiotics). Therefore, it can be seen that the combination of compound 1 with meropenem will make the treatment of bacterial infections easier, and has a wide clinical application prospects.

Test Example 2 Effect of compound 1 in combination with polymyxin against drug-resistant *Acinetobacter baumannii*

This experiment is designed to determine the minimum inhibitory concentration (MIC) of compound 1 in combination with polymyxin against carbapenem-resistant *Acinetobacter baumannii* and to calculate the fractional inhibitory concentration (FIC) index.

The materials and methods involved are as follows:

### 1. Test compound

| No. | Compound name | Batch number | Content wt% | Source |
|---|---|---|---|---|
| 1 | Compound 1 | DD20210033-08-P | 98.2 | Asieris Pharmaceuticals (Shanghai) Co., Ltd. |
| 2 | Polymyxin | 130327-200906 | 75.8 | China National Institutes for Food and Drug Control |

### 2. Strains

Test bacteria: 10 strains of carbapenem-resistant *Acinetobacter baumannii* are clinically isolated strains, and are provided by Shanghai Junji Medical Laboratory Co., Ltd.

### 3. Drug sensitivity test method

According to the recommendation of the Clinical and Laboratory Standards Institute (CLSI), the microbroth dilution method designed by the chessboard method was used to determine the minimum inhibitory concentration (MIC) of compound 1 in combination with polymyxin against the above strains, and the fractional inhibitory concentration (FIC) index was calculated.

### (1) Formulation of antimicrobial drugs and preparation of drug sensitivity plates

Compound 1 was dissolved in dimethyl sulfoxide to obtain a stock solution I with a concentration of 51200 mg/L. The stock solution I was subjected to 1:1 gradient dilution with dimethyl sulfoxide to obtain a dilution solution with a concentration range of 51200 to 25 mg/L. The corresponding concentrations were added to sterile water for 100-fold dilution to obtain working solution I with a concentration range of 512 to 0.25 mg/L.

Polymyxin was dissolved in sterile water to obtain a stock solution with a concentration of 5120 mg/L. The polymyxin stock solution was subjected to 1:1 gradient dilution with sterile water to obtain a working solution II with a concentration range of 512 to 0.25 mg/L.

According to the method in Example 1, a drug sensitive plate was prepared with working solution I and working solution II, and the specific process will not be repeated here.
(2) The culture medium of the bacterial solution: Cation-Adjusted Mueller-Hinton Broth (CAMHB), a product of BBL, USA, batch number 9015952.
(3) Formulation method of bacterial solution: According to the direct colony suspension method, the test bacteria cultured overnight were prepared into a 0.5 McFarland turbidity tube using physiological saline. Then, the resulting solution was dilute 100 times with the above culture medium. 100 µL of the resulting bacterial solution was mixed well with the solution (100 µL) in each well of the drug sensitivity plate, and the final inoculation amount was 10⁵ CFU/mL.
(4) Culture conditions: Culturing at 35±2°C for 20 hours in air.

### 4. Results and evaluation

(1) The experimental results were determined according to the standard of CLSI 2020 M100 30^{th} edition.
(2) Calculation formula of fractional inhibitory concentration index: FIC = MIC of drug A in combination/MIC of drug A alone + MIC of drug B in combination/MIC of drug B alone; wherein: FIC≤0.5 indicates synergistic effect; 0.5<FIC≤1.0 indicates additive effect; 1<FIC≤2.0 indicates irrelevant effect; and FIC>2 indicates antagonistic effect.

The specific experimental results are shown in Table 2 below, wherein the *Acinetobacter baumannii* specifically refers to "carbapenem-resistant *Acinetobacter baumannii".*

**Table 2 FIC index of compound 1 in combination with polymyxin**

| No. | Bacteria name | Strain No. | Compound 1 (mg/L) | | Polymyxin (mg/L) | | FIC index | Results |
|---|---|---|---|---|---|---|---|---|
| | | | MIC alone | MIC in combination | MIC alone | MIC in combination | | |
| 1 | *Acinetobacter baumannii* | 18-J11-034 | 4 | 1 | 0.5 | 0.125 | 0.500 | Synergistic effect |
| 2 | *Acinetobacter baumannii* | 18-J15-030 | 16 | 1 | 0.5 | 0.125 | 0.313 | Synergistic effect |
| 3 | *Acinetobacter baumannii* | 18-J21-095 | 8 | 4 | 0.25 | 0.06 | 0.740 | Additive effect |
| 4 | *Acinetobacter baumannii* | 18-J24-015 | 8 | 1 | 0.5 | 0.125 | 0.375 | Synergistic effect |
| 5 | *Acinetobacter baumannii* | 18-J24-033 | 4 | 1 | 0.5 | 0.125 | 0.500 | Synergistic effect |
| 6 | *Acinetobacter baumannii* | 18-J40-021 | 8 | 1 | 1 | 0.125 | 0.250 | Synergistic effect |
| 7 | *Acinetobacter baumannii* | 18-J51-052 | 4 | 2 | 0.5 | 0.125 | 0.750 | Additive effect |
| 8 | *Acinetobacter baumannii* | 18-J11-001 | 8 | 0.5 | 0.25 | 0.06 | 0.303 | Synergistic effect |
| 9 | *Acinetobacter baumannii* | 18-J11-097 | 4 | 0.5 | 0.25 | 0.06 | 0.365 | Synergistic effect |
| 10 | *Acinetobacter baumannii* | 18-J11-002 | 4 | 2 | 0.25 | 0.06 | 0.740 | Additive effect |

As can be seen from Table 2, the combination of compound 1 with polymyxin can significantly reduce the MIC of polymyxin, and shows a synergistic effect on carbapenem-resistant *Acinetobacter baumannii.* Therefore, it can be seen that the combination of compound 1 with meropenem will make the treatment of bacterial infections easier, and has a wide clinical application prospects.

### Test Example 3 Effect of compound 2 in combination with polymyxin against drug-resistant Acinetobacter baumannii

This experiment is designed to determine the minimum inhibitory concentration (MIC) of compound 2 in combination with polymyxin against carbapenem-resistant *Acinetobacter baumannii* and to calculate the fractional inhibitory concentration (FIC) index.

The materials and methods involved are as follows:

### 1. Test compound

| No. | Compound name | Batch number | Content wt% | Source |
|---|---|---|---|---|
| 1 | Compound 2 | DD033-21-1781 | 98.44 | Asieris Pharmaceuticals (Shanghai) Co., Ltd. |
| 2 | Polymyxin | 130327-200906 | 75.8 | China National Institutes for Food and Drug Control |

### 2. Strains

Test bacteria: 10 strains of carbapenem-resistant *Acinetobacter baumannii* are clinically isolated strains, and are provided by Shanghai Junji Medical Laboratory Co., Ltd.

### 3. Drug sensitivity test method

According to the recommendation of the Clinical and Laboratory Standards Institute (CLSI), the microbroth dilution method designed by the chessboard method was used to determine the minimum inhibitory concentration (MIC) of compound 2 in combination with polymyxin against the above strains, and the fractional inhibitory concentration (FIC) index was calculated.

### (1) Formulation of antimicrobial drugs and preparation of drug sensitivity plates

Compound 2 was dissolved in dimethyl sulfoxide to obtain a stock solution I with a concentration of 51200 mg/L. The stock solution I was subjected to 1:1 gradient dilution with dimethyl sulfoxide to obtain a dilution solution with a concentration range of 51200 to 25 mg/L. The corresponding concentrations were added to sterile water for 100-fold dilution to obtain working solution I with a concentration range of 512 to 0.25 mg/L.

Polymyxin was dissolved in sterile water to obtain a stock solution with a concentration of 5120 mg/L. The meropenem stock solution was subjected to 1:1 gradient dilution with sterile water to obtain a working solution II with a concentration range of 512 to 0.25 mg/L.

According to the method in Example 1, a drug sensitive plate was prepared with working solution I and working solution II, and the specific process will not be repeated here.
(2) The culture medium of the bacterial solution: Cation-Adjusted Mueller-Hinton Broth (CAMHB), a product of BBL, USA, batch number 9015952.
(3) Formulation method of bacterial solution: According to the direct colony suspension method, the test bacteria cultured overnight were prepared into a 0.5 McFarland turbidity tube using physiological saline. Then, the resulting solution was dilute 100 times with the above culture medium. 100 µL of the resulting bacterial solution was mixed well with the solution (100 µL) in each well of the drug sensitivity plate, and the final inoculation amount was 10⁵ CFU/mL.
(4) Culture conditions: Culturing at 35±2°C for 20 hours in air.

### 4. Results and evaluation

(1) The experimental results were determined according to the standard of CLSI 2020 M100 30^{th} edition.
(2) Calculation formula of fractional inhibitory concentration index: FIC = MIC of drug A in combination/MIC of drug A alone + MIC of drug B in combination/MIC of drug B alone; wherein: FIC≤0.5 indicates synergistic effect; 0.5<FIC≤1.0 indicates additive effect; 1<FIC≤2.0 indicates irrelevant effect; and FIC>2 indicates antagonistic effect.

The specific experimental results are shown in Table 3 below, wherein the *Acinetobacter baumannii* specifically refers to "carbapenem-resistant *Acinetobacter baumannii".*

**Table 3 FIC index of compound 2 in combination with polymyxin**

| No. | Bacteria name | Strain No. | Compound 2 (mg/L) | | Polymyxin (mg/L) | | FIC index | Results |
|---|---|---|---|---|---|---|---|---|
| | | | MIC alone | MIC in combination | MIC alone | MIC in combination | | |
| 1 | *Acinetobacter baumannii* | 18-J11-034 | 4 | 1 | 0.5 | 0.25 | 0.750 | Additive effect |
| 2 | *Acinetobacter baumannii* | 18-J15-030 | 4 | 1 | 0.5 | 0.25 | 0.750 | Additive effect |
| 3 | *Acinetobacter baumannii* | 18-J21-095 | 4 | 2 | 0.5 | 0.25 | 1.000 | Additive effect |
| 4 | *Acinetobacter baumannii* | 18-J24-015 | 4 | 1 | 1 | 0.25 | 0.500 | Synergistic effect |
| 5 | *Acinetobacter baumannii* | 18-J24-033 | 4 | 2 | 1 | 0.125 | 0.625 | Additive effect |
| 6 | *Acinetobacter baumannii* | 18-J40-021 | 4 | 1 | 1 | 0.25 | 0.500 | Synergistic effect |
| 7 | *Acinetobacter baumannii* | 18-J51-052 | 4 | 1 | 1 | 0.25 | 0.500 | Synergistic effect |
| 8 | *Acinetobacter baumannii* | 18-J11-001 | 8 | 1 | 0.5 | 0.125 | 0.375 | Synergistic effect |
| 9 | *Acinetobacter baumannii* | 18-J11-097 | 4 | 0.5 | 0.5 | 0.125 | 0.375 | Synergistic effect |
| 10 | *Acinetobacter baumannii* | 18-J11-002 | 4 | 1 | 1 | 0.25 | 0.500 | Synergistic effect |

As can be seen from Table 3, the combination of compound 2 with polymyxin can significantly reduce the MIC of polymyxin, and shows a synergistic effect or additive effect on carbapenem-resistant *Acinetobacter baumannii.* Therefore, it can be seen that the combination of compound 2 with polymyxin will make the treatment of bacterial infections easier, and has a wide clinical application prospects.

### Test Example 4 Effect of compound A in combination with meropenem against drug-resistant Escherichia coli

This experiment is designed to determine the minimum inhibitory concentration (MIC) of compound A in combination with meropenem against drug-resistant *Escherichia coli* I (a strain resistant to carbapenem antibiotics) and drug-resistant *Escherichia coli* II (a strain resistant to fluoroquinolone antibiotics, non-fluoroquinolone antibiotics, aminoglycoside antibiotics, glycopeptide antibiotics and carbapenem antibiotics), and to calculate the fractional inhibitory concentration (FIC) index.

The materials and methods involved are as follows:

### 1. Test compound

| No. | Compound name | Batch number | Content % | Source |
|---|---|---|---|---|
| 1 | Compound A | PC007-009-PlA | 99 | Asieris Pharmaceuticals (Shanghai) Co., Ltd. |
| 2 | Meropenem | 130506-201403 | 87 | China National Institutes for Food and Drug Control |

### 2. Strains

### Test bacteria:

3 strains of drug-resistant *Escherichia coli* I, namely drug-resistant *Escherichia coli* 18-WO9-093, drug-resistant *Escherichia coli* 18-W37-025, and drug-resistant *Escherichia coli* 18-W37-032, are provided by Shanghai Junji Medical Laboratory Co., Ltd. These 3 strains are all resistant to carbapenem antibiotics (e.g., meropenem).

1 strain of drug-resistant *Escherichia coli* II, namely drug-resistant *Escherichia coli* 1864, is provided by the School of Pharmacy of Fudan University. This strain is resistant to fluoroquinolone antibiotics (such as levofloxacin), non-fluoroquinolone antibiotics (such as nalidixic acid, nemofloxacin), aminoglycoside antibiotics (such as gentamicin), glycopeptide antibiotics (such as vancomycin) and carbapenem antibiotics (such as meropenem).

| No. | Drug type | Drug | MIC/(µg/mL) |
|---|---|---|---|
| 1 | Fluoroquinolone antibiotics | Levofloxacin | 32 |
| 2 | Non-fluoroquinolone antibiotics | Nalidixic acid | >64 |

| | | | |
|---|---|---|---|
| 3 | Non-fluoroquinolone antibiotics | Nemofloxacin | >64 |
| 4 | Aminoglycoside antibiotics | Gentamycin | 64 |
| 5 | Glycopeptide antibiotics | Vancomycin | >64 |
| 6 | Carbapenem antibiotics | Meropenem | >64 |

### 3. Drug sensitivity test method

According to the recommendation of the Clinical and Laboratory Standards Institute (CLSI), the microbroth dilution method designed by the chessboard method was used to determine the minimum inhibitory concentration (MIC) of compound A in combination with meropenem against the above strains, and the fractional inhibitory concentration (FIC) index was calculated.

### (1) Formulation of antimicrobial drugs and preparation of drug sensitivity plates

Compound A was dissolved in dimethyl sulfoxide to obtain a stock solution I with a concentration of 1280 mg/L. The stock solution I was diluted to 512 mg/L with sterile water to obtain a gradient dilution first tube solution I. The gradient dilution first tube solution I was subjected to 1: 1 gradient dilution with sterile water to obtain a working solution I with a gradient range of 512 to 0.25 mg/L.

Meropenem was dissolved in sterile water to obtain a stock solution II with a concentration of 5120 mg/L. The stock solution II was diluted to 1024 mg/L with sterile water to obtain a gradient dilution first tube solution II. The gradient dilution first tube solution II was subjected to 1:1 gradient dilution with sterile water to obtain a working solution II with a gradient range of 1024 to 0.5 mg/L.

According to the method in Example 1, a drug sensitive plate was prepared with working solution I and working solution II, and the specific process will not be repeated here.
(2) The culture medium of the bacterial solution: Cation-Adjusted Mueller-Hinton Broth (CAMHB), a product of BBL, USA, batch number 9015952.
(3) Formulation method of bacterial solution: According to the direct colony suspension method, the test bacteria cultured overnight were prepared into a 0.5 McFarland turbidity tube using physiological saline. Then, the resulting solution was dilute 100 times with the above culture medium. 100 µL of the resulting bacterial solution was mixed well with the solution (100 µL) in each well of the drug sensitivity plate, and the final inoculation amount was 10⁵ CFU/mL.
(4) Culture conditions: Culturing at 35±2°C for 20 hours in air.

### 4. Results and evaluation

(1) The experimental results were determined according to the standard of CLSI 2019 M100 29^{th} edition.
(2) Calculation formula of fractional inhibitory concentration index: FIC = MIC of drug A in combination/MIC of drug A alone + MIC of drug B in combination/MIC of drug B alone; wherein: FIC≤0.5 indicates synergistic effect; 0.5<FIC≤1.0 indicates additive effect; 1<FIC≤2.0 indicates irrelevant effect; and FIC>2 indicates antagonistic effect.

The specific experimental results are shown in Table 4 below.

**Table 4 FIC index of compound A in combination with meropenem**

| No. | Bacteria name | Strain No. | Compound A (mg/L) | | Meropenem (mg/L) | | FIC index | Results |
|---|---|---|---|---|---|---|---|---|
| | | | MIC alone | MIC in combination | MIC alone | MIC in combination | | |
| 1 | Drug-resistant *Escherichia coli I* | 18-WO9-093 | 32 | 8 | 256 | 2 | 0.258 | Synergistic effect |
| 2 | Drug-resistant *Escherichia coli I* | 18-W37-025 | 32 | 8 | 128 | 1 | 0.258 | Synergistic effect |
| 3 | Drug-resistant *Escherichia coli I* | 18-W37-032 | 32 | 8 | 128 | 4 | 0.281 | Synergistic effect |
| 4 | Drug-resistant *Escherichia coli* II | 1864 | 4 | 0.5 | 128 | 2 | 0.14 | Synergistic effect |

As can be seen from Table 4, the combination of compound A with meropenem can significantly reduce the MIC of meropenem, and shows a synergistic effect on drug-resistant *Escherichia coli* I (a strain resistant to carbapenem antibiotics) and drug-resistant *Escherichia coli* II (a strain resistant to fluoroquinolone antibiotics, non-fluoroquinolone antibiotics, aminoglycoside antibiotics, glycopeptide antibiotics and carbapenem antibiotics). Therefore, it can be seen that the combination of compound A with meropenem will make the treatment of bacterial infections easier, and has a wide clinical application prospects.

### Test Example 5 Effect of compound A in combination with meropenem against drug-resistant Acinetobacter baumannii

This experiment is designed to determine the minimum inhibitory concentration (MIC) of compound A in combination with meropenem against carbapenem-resistant *Acinetobacter baumannii* and to calculate the fractional inhibitory concentration (FIC) index.

The materials and methods involved are as follows:

### 1. Test compound

| No. | Compound name | Batch number | Content wt% | Source |
|---|---|---|---|---|
| 1 | Compound A | PC007-009-P1A | 99 | Asieris Pharmaceuticals (Shanghai) Co., Ltd. |
| 2 | Meropenem | 130506-202004 | 86.8 | China National Institutes for Food and Drug Control |

### 2. Strains

Test bacteria: 10 strains of carbapenem-resistant *Acinetobacter baumannii* are clinically isolated strains, and are provided by Shanghai Junji Medical Laboratory Co., Ltd.

### 3. Drug sensitivity test method

According to the recommendation of the Clinical and Laboratory Standards Institute (CLSI), the microbroth dilution method designed by the chessboard method was used to determine the minimum inhibitory concentration (MIC) of compound A in combination with meropenem against the above strains, and the fractional inhibitory concentration (FIC) index was calculated.

### (1) Formulation of antimicrobial drugs and preparation of drug sensitivity plates

Compound A was dissolved in dimethyl sulfoxide to obtain a stock solution I with a concentration of 51200 mg/L. The stock solution I was subjected to 1:1 gradient dilution with dimethyl sulfoxide to obtain a dilution solution with a concentration range of 51200 to 25 mg/L. The corresponding concentrations were added to sterile water for 100-fold dilution to obtain working solution I with a concentration range of 512 to 0.25 mg/L.

Meropenem was dissolved in sterile water to obtain a stock solution with a concentration of 5120 mg/L. The meropenem stock solution was subjected to 1:1 gradient dilution with sterile water to obtain a working solution II with a concentration range of 512 to 0.25 mg/L.

According to the method in Example 1, a drug sensitive plate was prepared with working solution I and working solution II, and the specific process will not be repeated here.
(2) The culture medium of the bacterial solution: Cation-Adjusted Mueller-Hinton Broth (CAMHB), a product of BBL, USA, batch number 9015952.
(3) Formulation method of bacterial solution: According to the direct colony suspension method, the test bacteria cultured overnight were prepared into a 0.5 McFarland turbidity tube using physiological saline. Then, the resulting solution was dilute 100 times with the above culture medium. 100 µL of the resulting bacterial solution was mixed well with the solution (100 µL) in each well of the drug sensitivity plate, and the final inoculation amount was 10⁵ CFU/mL.
(4) Culture conditions: Culturing at 35±2°C for 20 hours in air.

### 4. Results and evaluation

(1) The experimental results were determined according to the standard of CLSI 2020 M100 30^{th} edition.
(2) Calculation formula of fractional inhibitory concentration index: FIC = MIC of drug A in combination/MIC of drug A alone + MIC of drug B in combination/MIC of drug B alone; wherein: FIC≤0.5 indicates synergistic effect; 0.5<FIC≤1.0 indicates additive effect; 1<FIC≤2.0 indicates irrelevant effect; and FIC>2 indicates antagonistic effect.

The specific experimental results are shown in Table 5 below, wherein the *Acinetobacter baumannii* specifically refers to "carbapenem-resistant *Acinetobacter baumannii".*

**Table 5 FIC index of compound A in combination with meropenem**

| No. | Bacteria name | Compound A (mg/L) | | Meropenem (mg/L) | | FIC index | Results |
|---|---|---|---|---|---|---|---|
| | | MIC alone | MIC in combination | MIC alone | MIC in combination | | |
| 1 | | 32 | 0.06 | 128 | 16 | 0.127 | Synergistic effect |
| 2 | | 16 | 0.06 | 128 | 16 | 0.129 | Synergistic effect |
| 3 | | 16 | 0.06 | 128 | 16 | 0.129 | Synergistic effect |
| 4 | | 16 | 0.06 | 128 | 16 | 0.129 | Synergistic effect |
| 5 | *Acinetobacter baumannii* | 16 | 0.06 | 128 | 16 | 0.129 | Synergistic effect |
| 6 | | 16 | 0.06 | 128 | 16 | 0.129 | Synergistic effect |
| 7 | | 16 | 0.06 | 64 | 16 | 0.254 | Synergistic effect |
| 8 | | 8 | 0.06 | 64 | 8 | 0.133 | Synergistic effect |
| 9 | | 8 | 0.06 | 64 | 8 | 0.133 | Synergistic effect |
| 10 | | 16 | 0.06 | 128 | 16 | 0.129 | Synergistic effect |

As can be seen from Table 5, the combination of compound A with meropenem can significantly reduce the MIC of meropenem, and shows a synergistic effect on carbapenem-resistant *Acinetobacter baumannii.* Therefore, it can be seen that the combination of compound A with meropenem will make the treatment of bacterial infections easier, and has a wide clinical application prospects.

Although the specific embodiments of the present invention are described above, it should be understood by those skilled in the art that this is only for illustration and the protection scope of the present invention is defined by the appended claims. Those skilled in the art may make various changes or modifications to these embodiments without departing the principles and essence of the present invention, and these changes and modifications all fall within the protection scope of the present invention.

## Claims

1. A pharmaceutical composition comprising a compound of formula (I) or a pharmaceutically acceptable salt thereof and at least one antibacterial agent as active ingredients, and a pharmaceutically acceptable carrier or excipient, 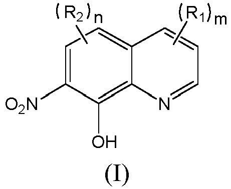 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is hydrogen;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is halogen;
n is 0, 1 or 2; and preferably 1 or 2;
m is 0, 1, 2 or 3; and preferably 0 or 1.

2. The pharmaceutical composition according to claim 1, wherein in the compound of formula (I), each R₁ is independently hydrogen, each R₂ is independently halogen, n is 1 or 2, and m is 1.

3. The pharmaceutical composition according to claim 1 or 2, wherein the compound of formula (I) is selected from the group consisting of:

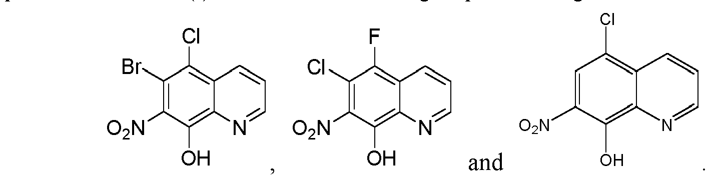

4. The pharmaceutical composition according to any one of claims 1 to 3, wherein the antibacterial agent is one or more selected from the group consisting of carbapenem antibiotics, polypeptide antibiotics, fluoroquinolone antibiotics, non-fluoroquinolone antibiotics, aminoglycoside antibiotics and glycopeptide antibiotics; the carbapenem antibiotic is for example meropenem, the polypeptide antibiotic is for example polymyxin, the fluoroquinolone antibiotic is for example levofloxacin, the non-fluoroquinolone antibiotic is for example nalidixic acid or nemonoxacin, the aminoglycoside antibiotic is for example gentamicin, the glycopeptide antibiotic is for example vancomycin; preferably the antibacterial agent is selected from the group consisting of carbapenem antibiotics and polypeptide antibiotics, and more preferably selected from the group consisting of polymyxin and meropenem.

5. The pharmaceutical composition according to any one of claims 1 to 4, wherein the pharmaceutical composition is an antibacterial pharmaceutical composition; the bacteria are preferably selected from strains of *Escherichia*; the strains of *Escherichia* are preferably *Escherichia coli,* wherein the *Escherichia coli* is non-resistant *Escherichia coli* and/or resistant *Escherichia coli,* preferably resistant *Escherichia coli,* and more preferably one or more of carbapenem-resistant *Escherichia coli,* quinolone-resistant *Escherichia coli,* aminoglycoside-resistant *Escherichia coli* and glycopeptide-resistant *Escherichia coli.*

6. The pharmaceutical composition according to any one of claims 1 to 4, wherein the pharmaceutical composition is an antibacterial pharmaceutical composition; the bacteria are preferably selected from strains of *Acinetobacter*; the strains of *Acinetobacter* are preferably *Acinetobacter baumannii,* wherein the *Acinetobacter baumannii* is non-resistant *Acinetobacter baumannii* and/or resistant *Acinetobacter baumannii,* preferably resistant *Acinetobacter baumannii,* and more preferably carbapenem-resistant *Acinetobacter baumannii.*

7. The pharmaceutical composition according to any one of claims 1 to 6, wherein the compound of formula (I) is 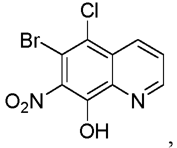 and the antibacterial agent is selected from the group consisting of meropenem and polymyxin.

8. The pharmaceutical composition according to any one of claims 1 to 6, wherein the compound of formula (I) is 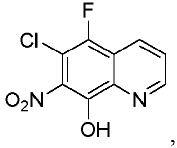 and the antibacterial agent is selected from the group consisting of polymyxin and meropenem, in particular polymyxin.

9. The pharmaceutical composition according to any one of claims 1 to 6, wherein the compound of formula (I) is 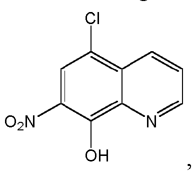 and the antibacterial agent is selected from the group consisting of polymyxin and meropenem, in particular meropenem.

10. Use of the pharmaceutical composition according to any one of claims 1 to 9 in the preparation of medicaments for the treatment of bacterial infectious diseases, especially multidrug-resistant bacterial infectious diseases.

11. Use of a compound of formula (I) or a pharmaceutically acceptable salt thereof or a pharmaceutical composition comprising the same in the preparation of medicaments for enhancing the antibacterial effect of an antibacterial agent, 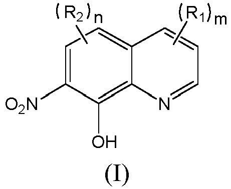 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is hydrogen;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is halogen;
n is 0, 1 or 2; and preferably 1 or 2;
m is 0, 1, 2 or 3; and preferably 0 or 1.

12. Use of a compound of formula (I) or a pharmaceutically acceptable salt thereof or a pharmaceutical composition comprising the same in combination with at least one antibacterial agent in the preparation of medicaments for enhancing the antibacterial effect of the antibacterial agent, 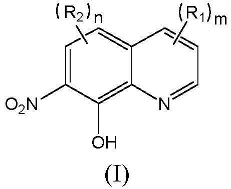 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is hydrogen;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is halogen;
n is 0, 1 or 2; and preferably 1 or 2;
m is 0, 1, 2 or 3; and preferably 0 or 1.

13. Use of a compound of formula (I) or a pharmaceutically acceptable salt thereof or a pharmaceutical composition comprising the same in combination with at least one antibacterial agent in the preparation of medicaments for the treatment of bacterial infectious diseases, especially multidrug-resistant bacterial infectious diseases, 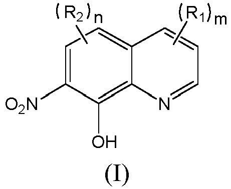 wherein:
each R₁ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is hydrogen;
each R₂ is independently selected from the group consisting of hydrogen, halogen, amino, hydroxy, thiol, cyano, nitro, oxo, alkyl, alkoxy, haloalkyl, haloalkoxy, hydroxyalkyl, aminoalkyl, cycloalkyl, heterocyclyl, aryl and heteroaryl; preferably selected from the group consisting of hydrogen, halogen, C₁₋₁₀ alkyl, C₁₋₁₀ alkoxy, C₁₋₁₀ haloalkyl, C₁₋₁₀ hydroxyalkyl, C₁₋₁₀ aminoalkyl, C₃₋₁₀ cycloalkyl, 3 to 10 membered heterocyclyl, C₆₋₁₄ aryl and 5 to 14 membered heteroaryl; more preferably selected from the group consisting of hydrogen, halogen, C₁₋₆ alkyl, C₁₋₆ alkoxy, C₁₋₆ haloalkyl, C₁₋₆ hydroxyalkyl, C₁₋₆ aminoalkyl, C₃₋₆ cycloalkyl, 3 to 6 membered heterocyclyl, C₆₋₁₀ aryl and 5 to 10 membered heteroaryl; further preferably selected from the group consisting of hydrogen, halogen, C₁₋₆ alkyl, C₁₋₆ alkoxy and C₁₋₆ haloalkyl; and most preferably is halogen;
n is 0, 1 or 2; and preferably 1 or 2;
m is 0, 1, 2 or 3; and preferably 0 or 1.

14. The use according to claim 12 or 13, wherein, in the compound of formula (I), each R₁ is independently hydrogen, each R₂ is independently halogen, n is 1 or 2, and m is 1.

15. The use according to any one of claims 12 to 14, wherein the compound of formula (I) is selected from the group consisting of:

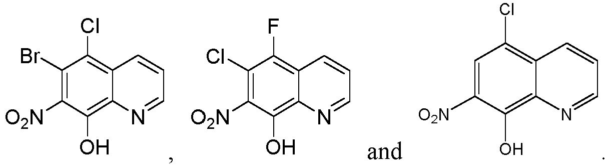

16. The use according to any one of claims 12 to 15, wherein the antibacterial agent is one or more selected from the group consisting of carbapenem antibiotics, polypeptide antibiotics, fluoroquinolone antibiotics, non-fluoroquinolone antibiotics, aminoglycoside antibiotics and glycopeptide antibiotics; the carbapenem antibiotic is for example meropenem, the polypeptide antibiotic is for example polymyxin, the fluoroquinolone antibiotic is for example levofloxacin, the non-fluoroquinolone antibiotic is for example nalidixic acid or nemonoxacin, the aminoglycoside antibiotic is for example gentamicin, the glycopeptide antibiotic is for example vancomycin; preferably the antibacterial agent is selected from the group consisting of carbapenem antibiotics and polypeptide antibiotics, and more preferably selected from the group consisting of polymyxin and meropenem.

17. The use according to any one of claims 14 to 16, wherein the bacteria are Gram-negative bacteria, such as *Escherichia coli, Pseudomonas aeruginosa* and *Acinetobacter baumannii*; preferably multidrug-resistant Gram-negative bacteria; more preferably multidrug-resistant bacteria resistant to carbapenem antibiotics, such as multidrug-resistant *Escherichia coli, Pseudomonas aeruginosa* and *Acinetobacter baumannii.*

18. The use according to any one of claims 12 to 17, wherein the compound of formula (I) is 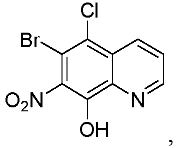 and the antibacterial agent is selected from the group consisting of meropenem and polymyxin.

19. The use according to any one of claims 12 to 17, wherein the compound of formula (I) is 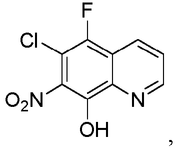 and the antibacterial agent is selected from the group consisting of polymyxin and meropenem, in particular polymyxin.

20. The use according to any one of claims 12 to 17, wherein the compound of formula (I) is 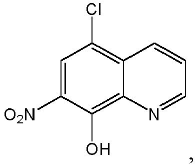 and the antibacterial agent is meropenem.

21. The use according to any one of claims 12 to 20, wherein the compound of formula (I) or the pharmaceutically acceptable salt thereof or the pharmaceutical composition comprising the same is administered simultaneously, successively or separately with the antibacterial agent.
